# EUROPÄISCHE PATENTANMELDUNG

(11) **EP 4 837 495 A1**
(43) Veröffentlichungstag der Anmeldung: **11.11.2026**
(21) Anmeldenummer: 25174611.1
(22) Anmeldetag: 06.05.2025
(51) Int. Cl.: A61B 1/00, A61B 1/018, A61B 1/267, A61B 17/00, A61B 1/015

(54) **OPERATIONSLARYNGOSKOP**

(71) Anmelder: Carl Reiner GmbH, 1090 Wien (AT)
(72) Erfinder: KÖLBL, Robert, 3430 Tulln (AT); LIRSCH, Dominik, 7100 Neusiedl am See (AT)
(74) Vertreter: SONN Patentanwälte GmbH & Co KG

(57) **Zusammenfassung**

Operationslaryngoskop (1), umfassend:
einen im Wesentlichen starren, länglichen Hauptkörper (2), der dazu eingerichtet ist, in die oberen Atemwege eines Patienten eingeführt zu werden, um einen Zugang zum Larynx, zur Trachea oder zu den Hauptbronchien zu schaffen;
wobei der Hauptkörper (2) mindestens einen durchgehenden Arbeitskanal definiert, der sich longitudinal durch den Hauptkörper (2) oder entlang einer seiner Oberflächen erstreckt und derart dimensioniert und ausgebildet ist, dass er die Passage eines oder mehrerer chirurgischer oder diagnostischer Instrumente durch den Hauptkörper (2) hindurch oder daran entlang bis in den Larynx, die Trachea oder die Hauptbronchien ermöglicht; und
einen Griff (3), der lösbar mit einem proximalen Ende (4) des Hauptkörpers (2) verbunden ist.

## Beschreibung

Die Erfindung betrifft ein Operationslaryngoskop, welches dazu dient, einen visuellen und instrumentellen Zugang zu den oberen Atemwegen eines Patienten, wie dem Larynx, der Trachea oder den Hauptbronchien, für chirurgische oder diagnostische Eingriffe zu schaffen.

Operationslaryngoskope sind wichtige Instrumente in der HNO-Chirurgie, der Thoraxchirurgie und der interventionellen Pneumologie. Sie umfassen einen starren Hauptkörper mit Arbeitskanal und, zur Manipulation des Instruments während der Einführung, Positionierung und des gesamten Eingriffs, einen Griff, der am proximalen, außerhalb des Patienten verbleibenden Ende des Hauptkörpers angeordnet ist.

Operationslaryngoskope sind von Anästhesie- oder Intubationslaryngoskopen zu unterscheiden. Während letztere primär der kurzzeitigen Visualisierung der Glottis dienen, um die endotracheale Intubation zu erleichtern und typischerweise einen Spatel zum Anheben der Epiglottis aufweisen, sind Operationslaryngoskope für interventionelle Zwecke konzipiert. Sie besitzen daher in der Regel einen im Wesentlichen starren, oft rohrförmigen oder distal offenen, rinnenförmigen Hauptkörper, der nicht nur Sicht verschafft, sondern vor allem einen geschützten und stabilen Arbeitskanal definiert. Dieser Arbeitskanal, der sich longitudinal durch den Hauptkörper oder entlang einer seiner Oberflächen erstreckt, ist derart dimensioniert und ausgebildet, dass er die Passage und Manipulation eines oder mehrerer chirurgischer oder diagnostischer Instrumente (z.B. Zangen, Scheren, Laserfasern, Absauger, Optiken) durch den Hauptkörper hindurch oder daran entlang bis in den Larynx, die Trachea oder die Hauptbronchien ermöglicht.

Bei den bekannten Operationslaryngoskopen ist dieser Griff üblicherweise fest und unlösbar mit dem Hauptkörper verbunden. Er kann integral mit dem Hauptkörper ausgebildet sein oder während des Herstellungsprozesses permanent daran befestigt werden, beispielsweise durch Schweißen oder eine andere unlösbare Verbindungstechnik. Zwar war es im Stand der Technik für Operationslaryngoskope teilweise üblich, bei der Fertigung auf spezifische Anwenderwünsche hinsichtlich der Griffgröße oder des Winkels, den der Griff relativ zur Längsachse des Hauptkörpers einnimmt, einzugehen. Das Ergebnis war jedoch stets ein spezifisches, monolithisches Instrument mit einer fixen, nachträglich nicht mehr änderbaren Griffkonfiguration für einen bestimmten Hauptkörper.

Der wesentliche Nachteil dieser bekannten Operationslaryngoskope liegt in ihrer mangelnden Flexibilität und Anpassungsfähigkeit im klinischen Einsatz, insbesondere angesichts der Vielfalt an Eingriffen und Patientenanatomien, für die sie verwendet werden. Unterschiedliche Patientenanatomien, die Art und Dauer des durchzuführenden Eingriffs oder auch die individuellen Präferenzen und die Handgröße des Operateurs können unterschiedliche Griffgrößen oder unterschiedliche Anstellwinkel des Griffs relativ zum Hauptkörper erfordern, um eine optimale, ermüdungsfreie Handhabung, eine stabile Positionierung des Hauptkörpers und eine gute Sicht auf das Operationsfeld über die gesamte Dauer des Eingriffs zu gewährleisten.

Da der Griff bei herkömmlichen Operationslaryngoskopen fest mit dem Hauptkörper verbunden ist, müssten Kliniken oder andere Anwender eine Vielzahl von kompletten Operationslaryngoskopen bevorraten, die sich lediglich durch die Griffkonfiguration (Größe und/oder Winkel), aber gegebenenfalls bei gleicher Größe und Form des Hauptkörpers unterscheiden, um für verschiedene Situationen und Anwender gerüstet zu sein. Dies ist nicht nur mit hohen Anschaffungs- und Lagerhaltungskosten verbunden, sondern erhöht auch den logistischen Aufwand für Bereitstellung, Reinigung und Sterilisation der zahlreichen Varianten erheblich. Die Alternative, nur wenige Varianten vorzuhalten, schränkt die Anpassungsfähigkeit an die jeweilige klinische Notwendigkeit oder Anwenderpräferenz empfindlich ein, was bei komplexen oder langwierigen Operationen nachteilig sein kann.

Der vorliegenden Erfindung liegt daher die Aufgabe zugrunde, ein Operationslaryngoskop der eingangs genannten Art bereitzustellen, das die Nachteile des Standes der Technik überwindet. Insbesondere soll ein Operationslaryngoskop geschaffen werden, das eine erhöhte Flexibilität und Anpassungsfähigkeit an unterschiedliche klinische Anforderungen und Anwenderpräferenzen ermöglicht, ohne dass eine Vielzahl kompletter Instrumente vorgehalten werden muss.

Zur Lösung dieser Aufgabe stellt die Erfindung ein Operationslaryngoskop bereit, umfassend:
einen im Wesentlichen starren, länglichen Hauptkörper, der dazu eingerichtet ist, in die oberen Atemwege eines Patienten eingeführt zu werden, um einen Zugang zum Larynx, zur Trachea oder zu den Hauptbronchien zu schaffen;
wobei der Hauptkörper mindestens einen durchgehenden Arbeitskanal definiert, der sich longitudinal durch den Hauptkörper oder entlang einer seiner Oberflächen erstreckt und derart dimensioniert und ausgebildet ist, dass er die Passage eines oder mehrerer chirurgischer oder diagnostischer Instrumente durch den Hauptkörper hindurch oder daran entlang bis in den Larynx, die Trachea oder die Hauptbronchien ermöglicht; und
einen Griff, der lösbar mit einem proximalen Ende des Hauptkörpers verbunden ist.

Der Kern der Erfindung liegt somit in der lösbaren Verbindung zwischen dem Hauptkörper und dem Griff des Operationslaryngoskops. Dieser Aufbau verleiht dem Instrument eine Modularität und damit eine signifikant erhöhte Flexibilität und Anpassungsfähigkeit. Anwender können nun einen einzigen Hauptkörper, der den für den Eingriff notwendigen Arbeitskanal bereitstellt, bedarfsgerecht mit verschiedenen Griffen kombinieren.

Diese Austauschbarkeit erlaubt die Wahl von Griffen unterschiedlicher Größe, um eine optimale Ergonomie für verschiedene Handgrößen oder zur Vermeidung von Ermüdung bei langen Eingriffen zu gewährleisten. Ebenso können Griffe mit unterschiedlicher Winkelorientierung zum Hauptkörper verwendet werden, um die Handhabung an spezifische Patientenanatomien oder die Anforderungen des chirurgischen Zugangs anzupassen.

Der daraus resultierende Vorteil ist eine erhebliche Effizienzsteigerung und Kostenreduktion. Statt einer Vielzahl kompletter Laryngoskope für jede mögliche Griffkonfiguration müssen lediglich eine Auswahl an Griffen und eine geringere Anzahl an Hauptkörpern vorgehalten werden. Dies reduziert die Anschaffungs-, Lager- und Sterilisationskosten und vereinfacht die Logistik. Zusätzlich kann die Trennbarkeit die Reinigung und Wartung der Komponenten erleichtern und ermöglicht bei Beschädigung den Austausch nur des betroffenen Teils, nicht des gesamten Instruments.

In einer bevorzugten Ausführungsform der Erfindung ist die lösbare Verbindung zwischen dem Griff und dem proximalen Ende des Hauptkörpers als formschlüssige Verbindung ausgebildet. Eine solche formschlüssige Verbindung gewährleistet eine hohe mechanische Stabilität und Steifigkeit, sodass Relativbewegungen wie Wackeln oder Verdrehen zwischen Griff und Hauptkörper im verbundenen Zustand wirksam unterbunden werden. Dies ist für die präzise Führung und Manipulation des Operationslaryngoskops während eines Eingriffs von großer Bedeutung.

Vorzugsweise weist hierfür ein Teil, entweder das proximale Ende des Hauptkörpers oder das distale Ende des Griffs, eine Aufnahmeausnehmung auf, die insbesondere als eine sich vorzugsweise in Längsrichtung des Hauptkörpers erstreckende Nut ausgebildet sein kann. Der jeweils andere Teil weist dann einen dazu komplementären Fortsatz auf, insbesondere eine entsprechend geformte Feder, die in die Aufnahmeausnehmung passt. Die Verbindung wird hergestellt, indem der Fortsatz bzw. die Feder in die Aufnahmeausnehmung bzw. die Nut eingeschoben wird, vorzugsweise durch eine geradlinige Bewegung entlang der Längsrichtung des Hauptkörpers. Diese Art der Verbindung bietet nicht nur eine definierte Ausrichtung der Komponenten zueinander, sondern nimmt auch effektiv Biege- und Torsionskräfte auf, was zu einer stabilen und sicheren Handhabung des gesamten Instruments führt.

Um die Sicherheit und Zuverlässigkeit der Verbindung zwischen Griff und Hauptkörper weiter zu erhöhen, umfasst das erfindungsgemäße Operationslaryngoskop in einer vorteilhaften Weiterbildung ferner einen Verriegelungsmechanismus. Dieser Mechanismus ist dazu eingerichtet, den Griff in seiner vollständig verbundenen Position am Hauptkörper lösbar zu sichern und somit ein unbeabsichtigtes Lösen der Verbindung während des Gebrauchs, beispielsweise durch Krafteinwirkung oder Vibration, zuverlässig zu verhindern.

Vorzugsweise weist dieser Verriegelungsmechanismus ein bewegliches Verriegelungselement auf. Dieses Verriegelungselement ist manuell oder durch die Verbindungsbewegung selbst zwischen mindestens zwei definierten Zuständen bewegbar: einer Entriegelungsposition, in welcher der Griff ungehindert vom Hauptkörper gelöst oder mit diesem verbunden werden kann, und einer Verriegelungsposition. In dieser Verriegelungsposition wirkt das Verriegelungselement form- oder kraftschlüssig mit einer korrespondierenden Struktur am jeweils anderen Bauteil (Griff oder Hauptkörper) zusammen und fixiert dadurch den Griff sicher relativ zum Hauptkörper gegen eine Lösungsbewegung.

Gemäß einer weiteren vorteilhaften Ausgestaltung der Erfindung ist das bewegliche Verriegelungselement am Griff angeordnet. In seiner Verriegelungsposition ist dieses Element dazu eingerichtet, die Trennebene zwischen dem Griff und dem proximalen Ende des Hauptkörpers zu überbrücken. Dabei wirkt es mit einem korrespondierenden Anschlag oder einer entsprechenden Verriegelungsgeometrie am Hauptkörper zusammen. Dieses Zusammenwirken ist derart gestaltet, dass es einer Bewegung des Griffs relativ zum Hauptkörper entgegenwirkt, die zu einem Lösen der Verbindung führen würde, beispielsweise einer axialen Abzugsbewegung entlang der Einführrichtung der formschlüssigen Verbindung. Auf diese Weise wird der Griff durch das am Griff befindliche Verriegelungselement, das in den Anschlag am Hauptkörper eingreift, sicher in seiner verbundenen und funktionsbereiten Position fixiert.

Besonders vorteilhaft für die Handhabung des Operationslaryngoskops im klinischen Alltag ist es, wenn das bewegliche Verriegelungselement derart angeordnet und ausgebildet ist, dass es manuell zwischen der Verriegelungs- und der Entriegelungsposition bewegt werden kann. Insbesondere ist es bevorzugt, dass die Betätigung des Verriegelungselements mit derselben Hand erfolgen kann, die auch den Griff hält. Dies kann beispielsweise durch eine geeignete Platzierung des Verriegelungselements am Griff oder Hauptkörper erreicht werden, sodass es leicht mit dem Daumen oder einem Finger der haltenden Hand erreichbar ist. Eine solche Ausgestaltung ermöglicht ein schnelles und intuitives Sichern bzw. Lösen des Griffs, ohne dass der Anwender umgreifen oder die zweite Hand zu Hilfe nehmen muss, was die Effizienz und Sicherheit bei der Anwendung erhöht.

In einer weiteren vorteilhaften Ausgestaltung ist das bewegliche Verriegelungselement derart gelagert und ausgebildet, dass es quer zur Einschieberichtung des Fortsatzes in die Aufnahmeausnehmung verschiebbar oder verschwenkbar ist. Diese quer zur Hauptverbindungsrichtung verlaufende Bewegung ermöglicht es dem Verriegelungselement, nach dem Herstellen der formschlüssigen Verbindung zwischen Griff und Hauptkörper, in seine Verriegelungsposition bewegt zu werden, ohne die primäre Verbindungsbewegung selbst zu behindern. In der Verriegelungsposition kann das Element dann beispielsweise einen Teil des anderen Bauteils (Griff oder Hauptkörper) hintergreifen, überdecken oder in eine entsprechende Ausnehmung eingreifen, um so eine Trennung der Verbindung entlang der ursprünglichen Einschieberichtung wirksam zu blockieren. Umgekehrt erlaubt die Bewegung des Elements in die Entriegelungsposition, ebenfalls quer zur Einschieberichtung, das problemlose Lösen des Griffs vom Hauptkörper.

Zur weiteren Verbesserung der Handhabung, insbesondere im Hinblick auf Reinigung und Wartung, ist in einer besonders vorteilhaften Ausgestaltung vorgesehen, dass das bewegliche Verriegelungselement lösbar am Griff angebracht ist. Das bedeutet, dass das Verriegelungselement als separates Bauteil konzipiert ist, das vom Griff entfernt und wieder daran befestigt werden kann, beispielsweise mittels geeigneter lösbarer Befestigungsmittel wie Schrauben oder einer Steck- und/oder Schnappverbindung. Diese Lösbarkeit bietet erhebliche Vorteile für die Reinigung und Sterilisation, da sowohl das Verriegelungselement selbst als auch der Bereich des Griffs, an dem es befestigt ist, gründlicher und einfacher gereinigt werden können. Schwer zugängliche Spalten werden so vermieden oder zumindest besser zugänglich gemacht. Des Weiteren ermöglicht diese Ausgestaltung eine einfache Wartung oder einen Austausch des Verriegelungselements im Falle von Verschleiß oder Beschädigung, ohne dass der gesamte Griff ausgetauscht werden muss, was die Lebensdauer des Instruments erhöht und die Servicefreundlichkeit verbessert.

Um die Verriegelungsposition des beweglichen Verriegelungselements eindeutig zu definieren und gegen unbeabsichtigtes Lösen aus dieser Position zu sichern, ist es weiterhin vorteilhaft, wenn das Verriegelungselement bei Erreichen der Verriegelungsposition mit korrespondierenden Rastmitteln zusammenwirkt. Diese Rastmittel sind so ausgelegt, dass das Verriegelungselement in der Verriegelungsposition einrastet. Dieses Einrasten kann beispielsweise durch eine kleine Erhebung am Verriegelungselement realisiert werden, die in eine komplementäre Vertiefung an dem Bauteil eingreift, relativ zu dem sich das Verriegelungselement bewegt (z.B. am Griff), oder umgekehrt. Das Einrasten bietet dem Anwender eine klare haptische und oft auch akustische Rückmeldung, dass die sichere Verriegelung erfolgt ist. Zudem wird das Verriegelungselement durch die Rastmittel stabil in der Verriegelungsposition gehalten, bis eine bewusste Kraft zur Überwindung der Rastung aufgebracht wird, um es gezielt in die Entriegelungsposition zu bewegen.

Um die Funktionalität des Operationslaryngoskops weiter zu erhöhen und den Anforderungen komplexer Eingriffe gerecht zu werden, ist es in einer vorteilhaften Ausgestaltung vorgesehen, dass der Hauptkörper ferner einen oder mehrere zusätzliche Kanäle aufweist. Diese zusätzlichen Kanäle sind separat vom primären Arbeitskanal für Instrumente ausgebildet und können für spezifische unterstützende Funktionen vorgesehen sein. Beispielsweise können solche Kanäle für die Absaugung von Sekreten, für die Spülung des Operationsfeldes oder für die Ventilation des Patienten dienen. Insbesondere kann ein dedizierter Kanal für spezielle Beatmungsformen, wie zum Beispiel die Jet-Ventilation, vorgesehen sein. Die Integration dieser zusätzlichen Kanäle direkt in den Hauptkörper rationalisiert den Eingriff, da weniger separate Instrumente eingeführt werden müssen, und verbessert die Übersichtlichkeit sowie die Effizienz während der Operation.

In einer vorteilhaften Ausgestaltung der Erfindung ist der zusätzliche Kanal ein Beatmungskanal. Ein solcher Beatmungskanal kann sich longitudinal durch den Hauptkörper oder entlang einer seiner Oberflächen erstrecken, und mündet typischerweise an oder nahe dem distalen Ende des Hauptkörpers. Vorzugsweise mündet der Beatmungskanal im Bereich des distalen Endes des Hauptkörpers in den

Arbeitskanal. Der primäre Zweck des Beatmungskanals ist es, die Zufuhr von Atemgasen, wie Sauerstoff oder Anästhesiegasgemischen, zum Patienten zu ermöglichen und somit die Oxygenierung und/oder Ventilation während des durch das Laryngoskop ermöglichten Eingriffs sicherzustellen oder zu unterstützen.

Besonders vorteilhaft ist es, wenn dieser zusätzliche Beatmungskanal spezifisch für die Durchführung einer Hochfrequenz-Jet-Beatmung (HFJV) ausgelegt ist. Die Jet-Beatmung ist eine Beatmungstechnik, bei der Gas unter hohem Druck intermittierend über eine Düse oder einen dünnen Katheter direkt in die Atemwege appliziert wird. Am proximalen Ende des Hauptkörpers weist der Beatmungskanal einen geeigneten Anschlussadapter (z.B. einen Luer-Lock-Anschluss oder einen spezifischen Jet-Ventilator-Anschluss) auf, der eine sichere und dichte Verbindung mit dem Schlauchsystem eines Beatmungsgeräts, wie z.B. eines Jet-Ventilators ermöglicht.

Für Anwendungsfälle, die eine besonders stabile Positionierung des Laryngoskops über einen längeren Zeitraum erfordern oder bei denen der Operateur beide Hände frei für die Instrumentenführung benötigt, ist es in einer weiteren vorteilhaften Ausgestaltung vorgesehen, dass der Hauptkörper Mittel zur Befestigung an einem externen Halterungssystem aufweist. Solche Halterungssysteme werden typischerweise für die Suspensionslaryngoskopie eingesetzt, bei der das Gewicht und die Positionierung des Laryngoskops durch einen mechanischen Arm oder eine andere Haltevorrichtung übernommen werden. Die am Hauptkörper vorgesehenen Mittel können beispielsweise spezielle Ausformungen, Nuten, Zapfen oder standardisierte Schnittstellen umfassen, die ein sicheres und zuverlässiges Einklemmen oder Ankoppeln an das Halterungssystem ermöglichen.

Hinsichtlich der für den Hauptkörper und den lösbaren Griff verwendeten Materialien müssen diese den üblichen Anforderungen an wiederverwendbare chirurgische Instrumente genügen. Dazu zählen insbesondere eine hohe Biokompatibilität, eine ausreichende mechanische Festigkeit und Steifigkeit zur Gewährleistung der Formstabilität während des Gebrauchs sowie eine hohe Beständigkeit gegenüber gängigen Sterilisationsverfahren, wie beispielsweise dem Autoklavieren. In einer bevorzugten Ausgestaltung der Erfindung sind sowohl der Hauptkörper als auch der lösbare Griff zumindest überwiegend, vorzugsweise vollständig, aus Titan oder einer Titanlegierung gefertigt. Die Wahl von Titan als Material für beide Komponenten bietet den Vorteil eines geringen spezifischen Gewichts bei gleichzeitig exzellenter Festigkeit und Korrosionsbeständigkeit.

Ein zweiter Aspekt der vorliegenden Erfindung betrifft ein Set für ein Operationslaryngoskop. Dieses Set umfasst zum einen ein Operationslaryngoskop gemäß dem ersten Aspekt der Erfindung, also einen Hauptkörper mit den beschriebenen Merkmalen und mindestens einen dazu passenden, lösbar verbindbaren Griff. Erfindungsgemäß umfasst das Set zusätzlich wenigstens einen weiteren Griff. Die Griffe innerhalb des Sets, also der erste und der mindestens eine weitere Griff, unterscheiden sich voneinander hinsichtlich mindestens eines Merkmals, das aus der Gruppe bestehend aus Griffgröße und Winkelorientierung des Griffs relativ zum Hauptkörper im verbundenen Zustand ausgewählt ist. Ein solches Set stellt dem Anwender unmittelbar die Kernvorteile der modularen Bauweise zur Verfügung: Es ermöglicht die flexible Auswahl des am besten geeigneten Griffs für die spezifische klinische Situation oder die Präferenz des Anwenders, ohne dass mehrere komplette Laryngoskope angeschafft und verwaltet werden müssen. Das Set maximiert somit die Anpassungsfähigkeit und Kosteneffizienz des Systems.

Die Erfindung wird nachfolgend anhand eines in der Zeichnung schematisch dargestellten Ausführungsbeispiels näher erläutert. Es zeigen:
Figur 1: Eine perspektivische Ansicht eines erfindungsgemäßen Operationslaryngoskops in einem demontierten Zustand, wobei der Hauptkörper und der Griff voneinander getrennt sind.
Figur 2: Eine perspektivische Ansicht des Operationslaryngoskops während des Verbindungsvorgangs des Griffs mit dem Hauptkörper.
Figur 3: Eine perspektivische Ansicht des Operationslaryngoskops in einem vollständig montierten, aber noch unverriegelten Zustand.
Figur 4: Eine perspektivische Ansicht des Operationslaryngoskops im montierten und verriegelten Zustand.
Figur 5: Den Griff in unterschiedlichen Größen.

Das Operationslaryngoskop 1 umfasst im Wesentlichen zwei Hauptkomponenten: einen länglichen, im Wesentlichen starren Hauptkörper 2 und einen Griff 3, der lösbar mit dem Hauptkörper 2 verbunden werden kann.

Der Hauptkörper 2 ist dazu eingerichtet, in die oberen Atemwege eines Patienten eingeführt zu werden. Er weist einen nicht explizit dargestellten, aber im Inneren verlaufenden Arbeitskanal auf, der die Passage von Instrumenten ermöglicht und sowohl am proximalen Ende 4 als auch am distalen Ende 5 offen ist. Der Hauptkörper 2 kann zudem wenigstens einen Beatmungskanal 6 aufweisen, der mit einem Anschluss 7 für ein Beatmungsgerät ausgestattet ist.

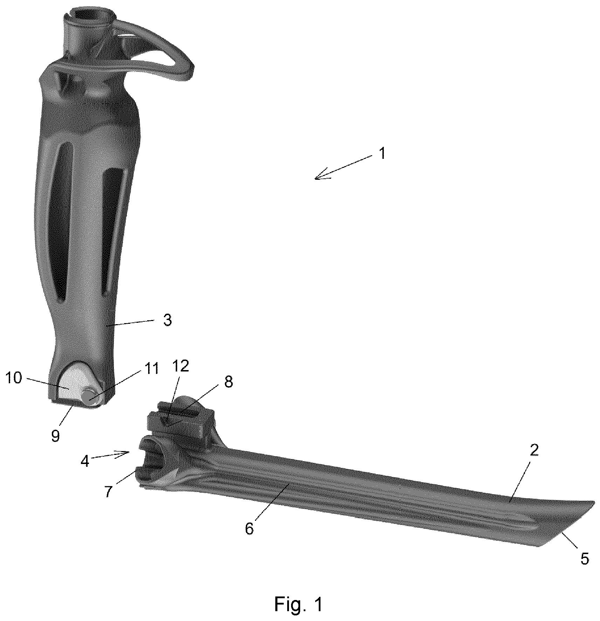

Wie insbesondere in Figur 1 ersichtlich, weist der Hauptkörper 2 an seinem proximalen Ende 4 eine Aufnahmeausnehmung 8 auf. Diese Aufnahmeausnehmung 8 ist im gezeigten Beispiel als nutartige Struktur ausgebildet und dient zur Aufnahme eines komplementären Teils des Griffs 3. Die Aufnahmeausnehmung 8 bzw. Nut weist vorzugsweise einen trapezförmigen Querschnitt auf.

Der Griff 3 dient der manuellen Handhabung und Manipulation des Operationslaryngoskops 1. An seinem im Gebrauch distalen Ende weist der Griff 3 einen Verbindungsfortsatz 9 auf, der komplementär zur Aufnahmeausnehmung 8 des Hauptkörpers 2 geformt ist und vorzugsweise ebenfalls einen trapezförmigen Querschnitt aufweisen kann. Im gezeigten Beispiel ist der Verbindungsfortsatz 9 als zungenartige Struktur ausgebildet, die passgenau in die Aufnahmeausnehmung 8 eingeschoben werden kann. Die Aufnahmeausnehmung 8 und der Verbindungsfortsatz 9 bilden zusammen eine formschlüssige Verbindung, die vorzugsweise als Schwalbenschwanzführung oder eine ähnliche Nut-Feder-Verbindung ausgestaltet sein kann, um eine hohe Stabilität gegen Biege- und Torsionskräfte zu gewährleisten.

Um die hergestellte Verbindung gegen unbeabsichtigtes Lösen zu sichern, ist ein Verriegelungsmechanismus vorgesehen. Dieser umfasst im gezeigten Ausführungsbeispiel ein bewegliches Verriegelungselement 10, das am Griff 3, genauer gesagt im Bereich des Verbindungsfortsatzes 9, angeordnet ist. Das Verriegelungselement 10 ist hier als ein kleines, um die Achse 10 schwenkbares Plättchen ausgebildet. Der Hauptkörper 2 weist eine korrespondierende Verriegelungsausnehmung 12 auf, beispielsweise eine Ausnehmung, einen Absatz oder eine Nut.

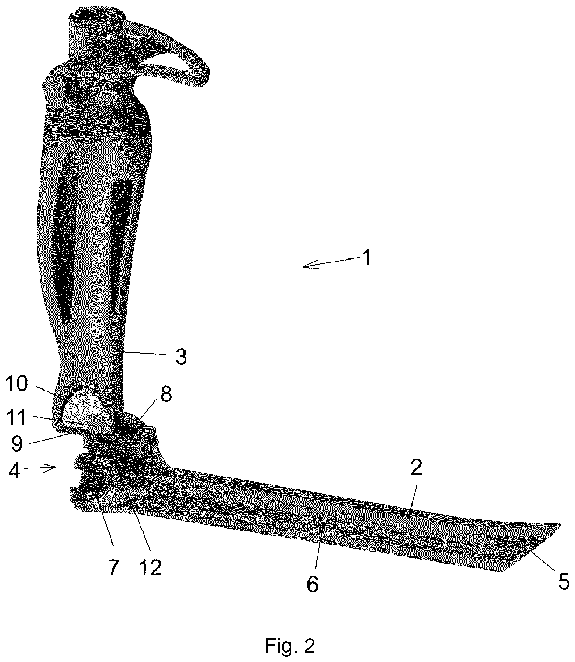

Figur 1 zeigt die getrennten Komponenten. Figur 2 illustriert den Vorgang des Verbindens, bei dem der Griff 3 mit seinem Verbindungsfortsatz 9 auf die Aufnahmeausnehmung 8 am proximalen Ende 7 des Hauptkörpers 2 ausgerichtet und anschließend, vorzugsweise durch eine geradlinige Bewegung entlang der Längsachse der Verbindung, eingeschoben wird. Das Verriegelungselement 10 befindet sich dabei in seiner Entriegelungsposition.

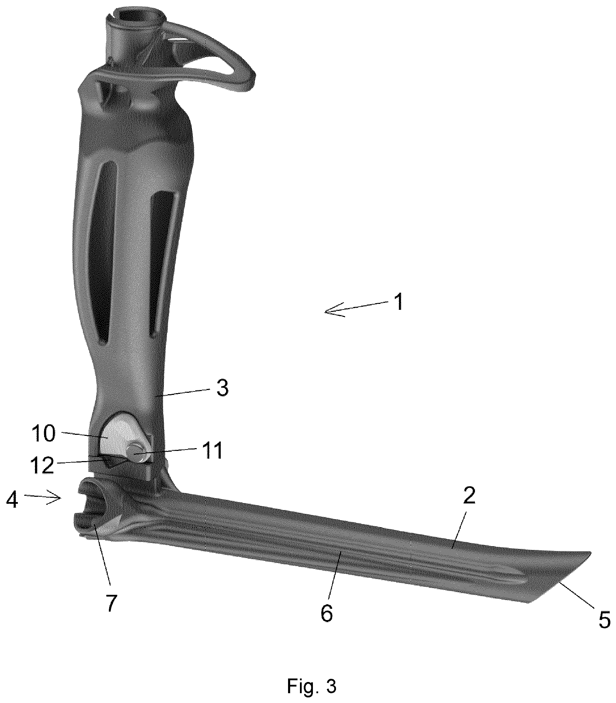

Figur 3 zeigt nun den Zustand, nachdem der Verbindungsfortsatz 9 vollständig in die Aufnahmeausnehmung 8 eingeschoben wurde. Der Griff 3 ist somit mechanisch mit dem Hauptkörper 2 verbunden. Das bewegliche Verriegelungselement 10 befindet sich in diesem Zustand jedoch noch in seiner Entriegelungs- oder einer Zwischenposition. Es greift noch nicht oder nicht vollständig in die Verriegelungsausnehmung 12 am Griff 3 ein, um eine sichere Verriegelung zu bewirken. Die Verbindung ist hergestellt, aber noch nicht gegen unbeabsichtigtes Lösen gesichert.

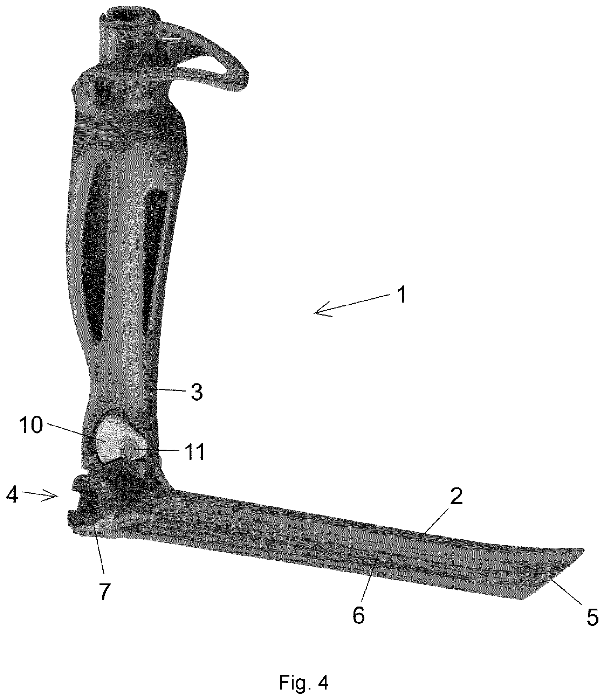

Figur 4 illustriert schließlich den vollständig gesicherten Zustand. Ausgehend vom Zustand in Figur 3 wurde das Verriegelungselement 10 nun manuell in seine Verriegelungsposition bewegt. In dieser Position greift das Verriegelungselement 10 formschlüssig in die Verriegelungsausnehmung 12 ein. Dadurch wird eine Relativbewegung zwischen Griff 3 und Hauptkörper 2, insbesondere eine Trennung entlang der Einschieberichtung, wirksam blockiert. Es können auch Rastmittel vorgesehen sein (nicht explizit gezeigt), die das Verriegelungselement 10 in der Verriegelungsposition mit einer spür- und/oder hörbaren Rückmeldung sichern.

Zum Lösen des Griffs 3 vom Hauptkörper 2 wird das Verriegelungselement 10 manuell zurück in seine Entriegelungsposition (wie in Fig. 3 gezeigt) bewegt, wodurch die Blockierung aufgehoben wird. Anschließend kann der Griff 3 einfach aus der Aufnahmeausnehmung 8 herausgezogen werden.

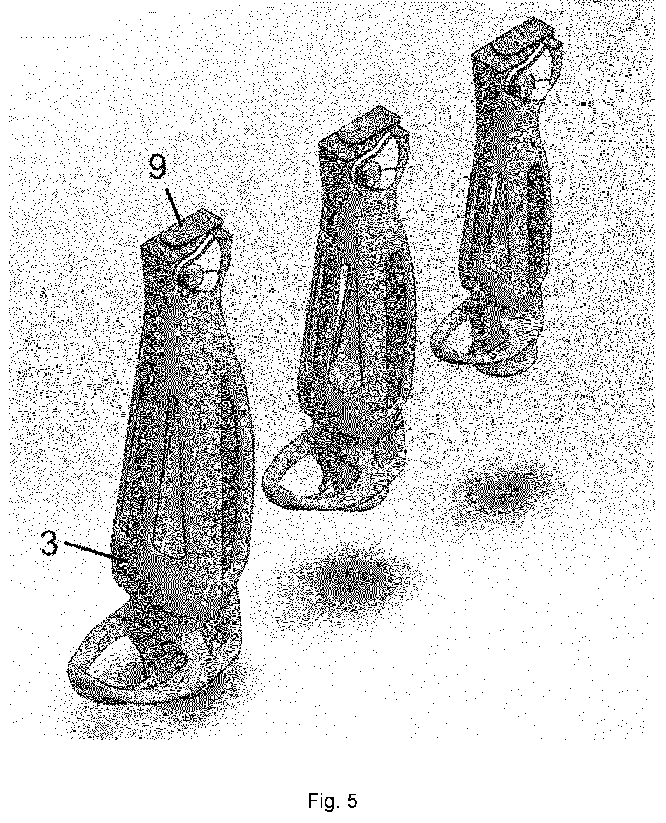

Figur 5 zeigt den Griff 3 in verschiedenen Größen.

## Patentansprüche

1. Operationslaryngoskop, umfassend:
einen im Wesentlichen starren, länglichen Hauptkörper (2), der dazu eingerichtet ist, in die oberen Atemwege eines Patienten eingeführt zu werden, um einen Zugang zum Larynx, zur Trachea oder zu den Hauptbronchien zu schaffen;
wobei der Hauptkörper (2) mindestens einen durchgehenden Arbeitskanal definiert, der sich longitudinal durch den Hauptkörper (2) oder entlang einer seiner Oberflächen erstreckt und derart dimensioniert und ausgebildet ist, dass er die Passage eines oder mehrerer chirurgischer oder diagnostischer Instrumente durch den Hauptkörper (2) hindurch oder daran entlang bis in den Larynx, die Trachea oder die Hauptbronchien ermöglicht; und
einen Griff (3), der lösbar mit einem proximalen Ende (4) des Hauptkörpers (2) verbunden ist.

2. Operationslaryngoskop nach Anspruch 1, **dadurch gekennzeichnet, dass** die lösbare Verbindung zwischen Griff und Hauptkörper (2) als formschlüssige Verbindung ausgebildet ist.

3. Operationslaryngoskop nach Anspruch 1 oder 2, **dadurch gekennzeichnet, dass** die lösbare Verbindung an einem Teil ausgewählt aus Hauptkörper (2) und Griff (3) eine vorzugsweise in Längsrichtung des Hauptkörpers (2) verlaufende Aufnahmeausnehmung (8), insbesondere Nut, aufweist und am anderen Teil ausgewählt aus Hauptkörper (2) und Griff (3) einen komplementären Fortsatz (9), insbesondere eine Feder, aufweist, wobei der Fortsatz (9) dazu eingerichtet ist, vorzugsweise in Längsrichtung des Hauptkörpers (2) in die Aufnahmeausnehmung (8) eingeschoben zu werden, um eine formschlüssige Verbindung herzustellen.

4. Operationslaryngoskop nach Anspruch 1, 2 oder 3, **dadurch gekennzeichnet, dass** ferner ein Verriegelungsmechanismus vorgesehen ist, der dazu eingerichtet ist, den Griff (3) in seiner verbundenen Position am Hauptkörper (2) lösbar zu sichern.

5. Operationslaryngoskop nach Anspruch 4, **dadurch gekennzeichnet, dass** der Verriegelungsmechanismus ein bewegliches Verriegelungselement (10) aufweist, das zwischen einer Entriegelungsposition, in der der Griff (3) vom Hauptkörper (2) gelöst werden kann, und einer Verriegelungsposition, in der das Verriegelungselement (10) den Griff (3) relativ zum Hauptkörper (2) fixiert, bewegbar ist.

6. Operationslaryngoskop nach Anspruch 5, **dadurch gekennzeichnet, dass** das bewegliche Verriegelungselement (10) am Griff (3) angeordnet ist und in der Verriegelungsposition eine Trennebene zwischen dem Griff (3) und dem Hauptkörper (2) überbrückend in eine entgegen einem Lösen der Verbindung wirkenden Anschlag (12) des Hauptkörpers (3) zusammenwirkt.

7. Operationslaryngoskop nach Anspruch 5 oder 6, **dadurch gekennzeichnet, dass** das bewegliche Verriegelungselement (10) derart angeordnet und ausgebildet ist, dass es manuell, insbesondere mit derselben Hand, die den Griff (3) hält, zwischen der Verriegelungs- und der Entriegelungsposition bewegt werden kann.

8. Operationslaryngoskop nach Anspruch 5, 6 oder 7, **dadurch gekennzeichnet, dass** das bewegliche Verriegelungselement (10) quer zur Einschieberichtung des Fortsatzes (9) in die Aufnahmeausnehmung (8) verschieb- oder veschwenkbar ist.

9. Operationslaryngoskop nach einem der Ansprüche 5 bis 8, **dadurch gekennzeichnet, dass** das bewegliche Verriegelungselement (10) lösbar am Griff (3) angebracht ist.

10. Operationslaryngoskop nach einem der Ansprüche 5 bis 9, **dadurch gekennzeichnet, dass** das Verriegelungselement (10) bei Erreichen der Verriegelungsposition mit Rastmitteln zusammenwirkt, sodass das Verriegelungselement (10) in der Verriegelungsposition einrastet.

11. Operationslaryngoskop nach einem der Ansprüche 1 bis 10, **dadurch gekennzeichnet, dass** der Hauptkörper (2) ferner einen oder mehrere zusätzliche Kanäle (6) für Absaugung, Spülung oder Ventilation, wie z.B. für Jet-Ventilation, aufweist.

12. Operationslaryngoskop nach einem der Ansprüche 1 bis 11, **dadurch gekennzeichnet, dass** der Hauptkörper (2) Mittel zur Befestigung an einem Halterungssystem für die Suspensionslaryngoskopie aufweist.

13. Set umfassend ein Operationslaryngoskop (1) nach einem der Ansprüche 1 bis 12, und wenigstens einen weiteren Griff (3), wobei sich die Griffe (3) voneinander hinsichtlich mindestens eines Merkmals ausgewählt aus der Gruppe bestehend aus Griffgröße und Winkelorientierung des Griffs (3) relativ zum Hauptkörper (2) im verbundenen Zustand unterscheiden.
